(19) Europäisches Patentamt / European Patent Office / Office européen des brevets

(11) **EP 2 929 937 A1**

(12) **EUROPEAN PATENT APPLICATION**

(43) Date of publication:
14.10.2015 Bulletin 2015/42

(51) Int Cl.:
*B01J 23/835* [(2006.01)]   *B01J 23/825* [(2006.01)]
*B01J 23/755* [(2006.01)]   *B01J 23/89* [(2006.01)]
*B01J 35/00* [(2006.01)]   *B01J 37/03* [(2006.01)]
*B01J 37/02* [(2006.01)]   *B01J 37/08* [(2006.01)]
*B01J 37/18* [(2006.01)]   *C10G 45/52* [(2006.01)]
*C10G 45/48* [(2006.01)]   *C07C 5/10* [(2006.01)]

(21) Application number: 15161805.5

(22) Date of filing: 31.03.2015

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **31.03.2014 JP 2014072648**

(71) Applicant: **JX Nippon Oil & Energy Corporation
Chiyoda-ku
Tokyo 100-8162 (JP)**

(72) Inventors:
• **Hirano, Yuichiro
Tokyo, Tokyo 100-8162 (JP)**
• **Furuta, Satoshi
Tokyo, Tokyo 100-8162 (JP)**

(74) Representative: **Grünecker Patent- und
Rechtsanwälte
PartG mbB
Leopoldstraße 4
80802 München (DE)**

(54) **Hydrogenation catalyst for aromatic hydrocarbon, and method for producing cyclic saturated hydrocarbon**

(57)   A hydrogenation catalyst for an aromatic hydrocarbon capable of inhibiting generation of a byproduct is provided. A hydrogenation catalyst for an aromatic hydrocarbon according to one aspect of the present invention comprises an active component containing an active metal element and an additional element, the active metal element is one selected from the group consisting of nickel, palladium and platinum, the additional element is one selected from the group consisting of tin, germanium, gallium, copper and iron, and an X-ray diffraction spectrum of the active component has a local maximum value at a diffraction angle da different from the diffraction angle dm, where a diffraction angle of diffracted X-ray derived from a crystal structure of a simple substance m of the active metal element is dm.

*Fig.2*

**Description**

TECHNICAL FIELD

The present invention relates to a hydrogenation catalyst for an aromatic hydrocarbons and a method for producing a cyclic saturated hydrocarbon using the same.

BACKGROUND

In recent years, it is expected to use, as a power source of a vehicle or the like, a fuel cell using hydrogen having a low environmental load as a fuel. In the processes of transportation, storage and supply of hydrogen, organic hydrides such as methylcyclohexane are utilized.

The organic hydrides refer to cyclic saturated hydrocarbons capable of reversibly repeating desorption and absorption of hydrogen by a dehydrogenation reaction and a hydrogenation reaction. The organic hydrides are in the form a liquid under ordinary temperature and pressure, have a smaller volume than a hydrogen gas, and are less reactive and safer than a hydrogen gas. Therefore, the organic hydrides are suitable for transportation and storage as compared with a simple substance of a hydrogen gas.

For example, in hydrogen producing facilities (such as a photovoltaic power plant), methylcyclohexane, which is one of the organic hydrides, is generated through hydrogenation of toluene, which is one of aromatic hydrocarbons. The methylcyclohexane is transported to a hydrogen consumption place, or stored in the consumption place. In the consumption place, hydrogen and toluene are generated by dehydrogenation of the methylcyclohexane. The thus generated hydrogen is supplied to a fuel cell. The toluene may be reused for generating methylcyclohexane by the hydrogenation in the hydrogen producing facilities.

As a hydrogenation catalyst for aromatic hydrocarbons, for example, an amorphous alloy catalyst containing nickel and aluminum as essential components is known (see Patent Literature 1).

Citation List

Patent Literature

Patent Literature 1: JP2002-542928

SUMMARY

If the hydrogenation reaction of an aromatic hydrocarbon is performed by using a conventional hydrogenation catalyst, however, not only hydrogenation of an aromatic site but also a carbon-carbon bond cleavage reaction such as demethylation may proceed to produce a byproduct in some cases.

The present invention was accomplished in consideration of the above-described problem of the conventional technique, and an object is to provide a hydrogenation catalyst for an aromatic hydrocarbon capable of inhibiting generation of a byproduct and a method for producing a cyclic saturated hydrocarbon using the same.

A hydrogenation catalyst for an aromatic hydrocarbon according to one aspect of the present invention comprises an active component containing an active metal element and an additional element, the active metal element is one selected from the group consisting of nickel, palladium and platinum, the additional element is one selected from the group consisting of tin, germanium, gallium, copper and iron, and an X-ray diffraction spectrum of the active component has a local maximum value at a diffraction angle da different from the diffraction angle dm, where a diffraction angle of diffracted X-ray derived from a crystal structure of a simple substance m of the active metal element is dm.

In the hydrogenation catalyst for an aromatic hydrocarbon according to one aspect of the present invention, M1 and M2 may satisfy the following expression (1):

$$0.01 \le M2/(M1 + M2) \le 0.6 \quad (1)$$

where a mole number of the active metal element contained in the active component is M1 and a mole number of the additional element contained in the active component is M2.

In the hydrogenation catalyst for an aromatic hydrocarbon according to one aspect of the present invention, the active metal element may be nickel, and the additional element may be tin.

The hydrogenation catalyst for an aromatic hydrocarbon according to one aspect of the present invention may further comprise silica on which the active component is supported.

A method for producing a cyclic saturated hydrocarbon according to one aspect of the present invention comprises a step of hydrogenating an aromatic hydrocarbon in the presence of the above-described hydrogenation catalyst and hydrogen.

According to the present invention, a hydrogenation catalyst for an aromatic hydrocarbon capable of inhibiting generation of a byproduct and a method for producing a cyclic saturated hydrocarbon using the same are provided.

BRIEF DESCRIPTION OF THE DRAWINGS

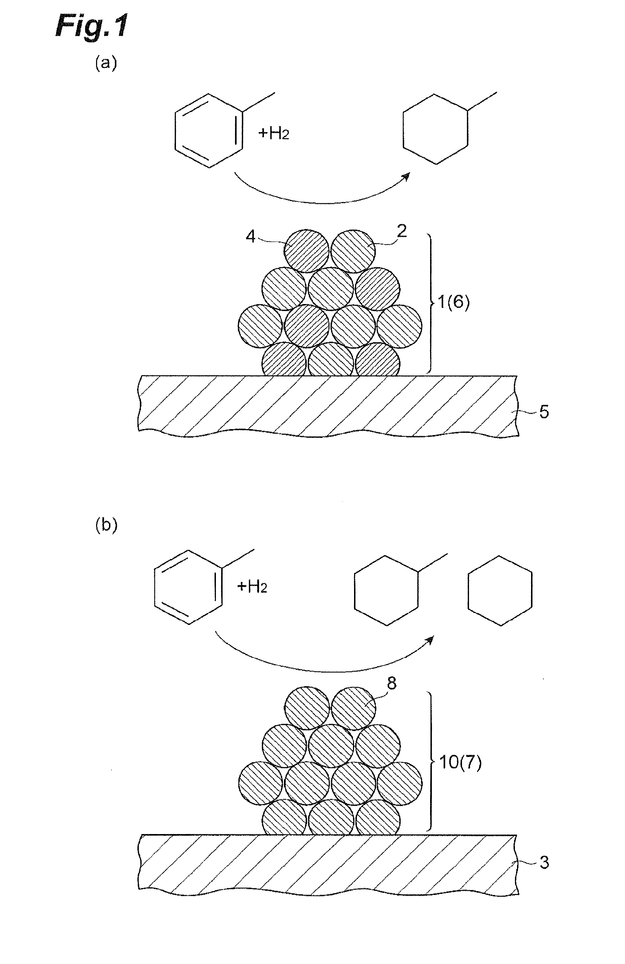

a in FIG. 1 (FIG. 1(a)) is a schematic diagram illustrating a surface of a hydrogenation catalyst according to an embodiment;
b in FIG. 1 (FIG. 1(b)) is a schematic diagram illustrating a surface of a conventional hydrogenation catalyst;
FIG. 2 is an X-ray diffraction (XRD) spectra of hydrogenation catalysts A-1 to A-4 and B-3 in a range of a diffraction angle of 35 to 55°;
FIG. 3 is a graph of a molar content of tin in an active component obtained when a hydrogenation catalyst is brought into contact with toluene, a toluene conversion rate and a cyclohexane concentration in a product oil by a hydrogenation reaction;
a in FIG. 4 (FIG. 4(a)) is an image of the hydrogenation catalyst A-1 taken by high-angle annular dark-field scanning transmission electron microscopy (HAADF-STEM);
b in FIG. 4 (FIG. 4(b)) is an image of a nickel distribution in the hydrogenation catalyst A-1 of FIG. 4(a);
c in FIG. 4 (FIG. 4(c)) is an image of a tin distribution in the hydrogenation catalyst A-1 of FIG. 4(a);
a in FIG. 5 (FIG. 5(a)) is another image of the hydrogenation catalyst A-1 taken by the HAADF-STEM;
b in FIG. 5 (FIG. 5(b)) is an image of a nickel distribution in the hydrogenation catalyst A-1 of FIG. 5(a);
c in FIG. 5 (FIG. 5(c)) is an image of a tin distribution in the hydrogenation catalyst A-1 of FIG. 5(a);
a in FIG. 6 (FIG. 6(a)) is another image of the hydrogenation catalyst A-1 taken by the HAADF-STEM;
b in FIG. 6 (FIG. 6(b)) is an image of a nickel distribution in the hydrogenation catalyst A-1 of FIG. 6(a);
c in FIG. 6 (FIG. 6(c)) is an image of a tin distribution in the hydrogenation catalyst A-1 of FIG. 6(a);
a in FIG. 7 (FIG. 7(a)) is an image of the hydrogenation catalyst A-2 taken by the HAADF-STEM;
b in FIG. 7 (FIG. 7(b)) is an image of a nickel distribution in the hydrogenation catalyst A-2 of FIG. 7(a);
c in FIG. 7 (FIG. 7(c)) is an image of a tin distribution in the hydrogenation catalyst A-2 of FIG. 7(a);
a in FIG. 8 (FIG. 8(a)) is another image of the hydrogenation catalyst A-2 taken by the HAADF-STEM;
b in FIG. 8 (FIG. 8(b)) is an image of a nickel distribution in the hydrogenation catalyst A-2 of FIG. 8(a);
c in FIG. 8 (FIG. 8(c)) is an image of a tin distribution in the hydrogenation catalyst A-2 of FIG. 8(a);
a in FIG. 9 (FIG. 9(a)) is another image of the hydrogenation catalyst A-2 taken by the HAADF-STEM;
b in FIG. 9 (FIG. 9(b)) is an image of a nickel distribution in the hydrogenation catalyst A-2 of FIG. 9(a); and
c in FIG. 9 (FIG. 9(c)) is an image of a tin distribution in the hydrogenation catalyst A-2 of FIG. 9(a).

A preferred embodiment of the present invention will now be described. It is noted that the present invention is not limited to the following embodiment at all.

DETAILED DESCRIPTION

<Conventional hydrogenation catalyst for aromatic hydrocarbons>

FIG. 1(b) is a schematic diagram of a surface of a conventional hydrogenation catalyst for aromatic hydrocarbons. The conventional hydrogenation catalyst 10 comprises, for example, one kind of active metal element 8 selected from nickel, palladium and platinum, and a support 3 on which the active metal element 8 is supported. The conventional hydrogenation catalyst 10 has a plurality of active sites 7 each containing the active metal element 8.

A chemical reaction by a catalyst can be classified into a structure-insensitive reaction and a structure-sensitive reaction. In the structure-insensitive reaction, the reaction rate is determined by merely the number of metal atoms exposed on a surface. In other words, the structure-insensitive reaction is a reaction not depending on the surface structure of a catalyst. A hydrogenation reaction of an aromatic hydrocarbon is the structure-insensitive reaction. On the other hand, in the structure-sensitive reaction, the reaction rate depends on the surface structure of a catalyst. For example, a carbon-carbon bond cleavage reaction such as demethylation proceeds through a mutual action between an active metal element constituting a prescribed steric structure in an active site (an active spot) of the catalyst and a carbon atom belonging to a methyl group or the like. In other words, it is presumed that the carbon-carbon bond cleavage reaction such as demethylation is the structure-sensitive reaction.

If the conventional hydrogenation catalyst 10 is used for performing, for example, a toluene hydrogenation reaction, a hydrogenation reaction of the aromatic hydrocarbon proceeds to generate methylcyclohexane, and in addition, a carbon-carbon bond cleavage reaction proceeds to eliminate a methyl group from the methylcyclohexane so as to generate cyclohexane as a byproduct. In other words, in the hydrogenation reaction using the conventional hydrogenation catalyst 10, not only the structure-insensitive reaction but also the structure-sensitive reaction occurs.

<Hydrogenation catalyst for an aromatic hydrocarbon of present embodiment>

The present inventors presumed that it is significant to highly disperse an active metal element in every active site constituted by an active component of a hydrogenation catalyst in order to inhibit the proceeding of a carbon-carbon bond cleavage reaction, which is a structure-sensitive reaction, resulting in accomplishing a hydrogenation catalyst of the present invention.

A hydrogenation catalyst for an aromatic hydrocarbon according to the present embodiment comprises an active component containing an active metal element and an additional element. The active component may contain the active metal element and the additional element alone. The hydrogenation catalyst has a plurality of active sites each constituted by the active component, and an aromatic hydrocarbon is hydrogenated in each of the active sites.

The active metal element is one selected from the group consisting of nickel, palladium and platinum. Such an active metal element has sufficient hydrogenation activity. The active metal element may be nickel that is excellent in the hydrogenation activity for aromatic hydrocarbons and is inexpensive and easily available.

The additional element of the hydrogenation catalyst of the present embodiment is one selected from the group consisting of tin, germanium, gallium, copper and iron. Such an additional element can disturb the crystal structure of a simple substance of the active metal element so as to highly disperse the active metal element. One selected from the group consisting of tin, germanium and gallium, and tin in particular, can easily highly disperse the active metal element.

An X-ray diffraction spectrum of the active component (or the hydrogenation catalyst) has a local maximum value at a diffraction angle da different from the diffraction angle dm, where a diffraction angle of diffracted X-ray derived from the crystal structure of the simple substance m of the active metal element is dm. An X-ray diffraction intensity Ia of the active component at the diffraction angle da is peculiar to the active component. Although the X-ray diffraction intensity Ia is the local maximum value in the X-ray diffraction spectrum of the active component, it is not necessarily the global maximum value in the X-ray diffraction spectrum of the active component. The X-ray diffraction spectrum of the active component may have a plurality of local maximum values at a plurality of diffraction angles da different from the diffraction angle dm. An X-ray diffraction intensity Im of the active component at the diffraction angle dm is derived from the crystal structure of the simple substance m of the active metal element that the hydrogenation catalyst can contain. The active component may contain the crystal of the simple substance m of the active metal element. The active component may not contain the crystal of the simple substance m of the active metal element. If the X-ray diffraction intensity Ia of the active component is larger than the X-ray diffraction intensity Im of the active component, the generation of a byproduct is easily inhibited in the hydrogenation reaction of aromatic hydrocarbons using the hydrogenation catalyst. The range of a diffraction angle $2\theta$ of the X-ray diffraction spectrum of the active component may be, for example, 5 to 90°. If the active component contains nickel and tin, the range of the diffraction angle $2\theta$ of the X-ray diffraction spectrum of the active component may be, for example, 35 to 55°. If the active component contains nickel and tin, the diffraction angle dm may be, for example, approximately 44.5° (44.51°), and the diffraction angle da may be, for example, approximately 43° (43.5°). If the active component contains platinum as the active metal element, the diffraction angle dm may be, for example, approximately 40.0° (39.8°). If the active component contains palladium as the active metal element, the diffraction angle dm may be, for example, approximately 40.0° (40.3°). If the active component contains an oxide of palladium, the diffraction angle dm may be, for example, approximately 33°.

The present inventors presume that the hydrogenation catalyst having the aforementioned technical characteristics on the X-ray diffraction spectrum of the active component has the following structure:

The X-ray diffraction intensity Ia is derived from the structure of a microcrystal of an alloy of the active component, the structure of a microcrystal of an intermetallic compound of the active component, or the structure of a microcrystal of a solid solution of the active component. In other words, the X-ray diffraction intensity Ia is derived from a regular structure constituted by the active component, and the active component is not always amorphous. The microcrystal of the alloy of the active component, the microcrystal of the intermetallic compound of the active component or the microcrystal of the solid solution of the active component is much smaller than a macro-crystal of the simple substance m. The X-ray diffraction spectrum of the active component having the local maximum value at the diffraction angle da different from the diffraction angle dm suggests that the macro-crystal structure of the simple substance m of the active metal element is disturbed, in each active site of the hydrogenation catalyst, by the interposition of the additional element to form the microcrystal of the alloy of the active component, the microcrystal of the intermetallic compound of the active component or the microcrystal of the solid solution of the active component, so as to disperse a large number of microcrystals in the active site.

FIG. 1(a) is a schematic diagram illustrating a part of a surface of the hydrogenation catalyst of the present embodiment. The hydrogenation catalyst 1 has a plurality of active sites 6. Each active site 6 is constituted by the alloy, the intermetallic compound or the solid solution of the active component. The active metal element 2 and the additional element 4 may be exposed on the surface of the active site 6. The active metal element 2 may be an atom or a molecule constituting the alloy, the intermetallic compound or the solid solution. The additional element 4 may be an atom or a molecule constituting the alloy, the intermetallic compound or the solid solution. In each active site 6 (in particular, on the surface of each active site 6), the additional element 4 intervenes between the active metal elements 2, and the active metal element 2 and the additional element 4 are highly dispersed.

If the hydrogenation catalyst having the above-described structure is used for performing, for example, a toluene hydrogenation reaction, a carbon-carbon bond cleavage reaction accompanying the hydrogenation reaction is inhibited, so as to improve selectivity of methylcyclohexane. Specifically, in the hydrogenation reaction of an aromatic hydrocarbon using the hydrogenation catalyst of the present embodiment, since the structure-sensitive reaction is inhibited and the structure-insensitive reaction can easily proceed, the generation of a byproduct is inhibited, and the aromatic hydrocarbon is selectively converted into a desired cyclic saturated hydrocarbon.

The mole numbers M1 and M2 may satisfy the following expression (1):

$$0.01 \leq M2/(M1 + M2) \leq 0.6 \quad (1)$$

where the mole number of the active metal element 2 contained in the active component is M1 and the mole number of the additional element 4 contained in the active component is M2.

If M2/(M1 + M2) is 0.01 to 0.6, the generation of a byproduct can be easily inhibited, and there is a tendency that the aromatic hydrocarbon can be easily selectively converted into a desired cyclic saturated hydrocarbon. M2/(M1 + M2) may be 0.05 or more, 0.1 or more, 0.13 or more, or 0.23 or more. M2(M1 + M2) may be 0.56 or less, or 0.40 or less.

Each active site 6 may be constituted by a microcrystal (such as a crystal of an alloy, an intermetallic compound or a solid solution) containing the active metal element 2 and the additional element 4. A longest diameter of each active site 6 or a longest diameter of the microcrystal may be 2 to 50 nm. If the longest diameter of the active site 6 or the microcrystal falls in the above-described range, the generation of a byproduct is easily inhibited, and there is a tendency that the aromatic hydrocarbon can be easily selectively converted into a desired cyclic saturated hydrocarbon.

The hydrogenation catalyst 1 may further comprise a support 5 on which the active component is supported. The support 5 may be, for example, silica, alumina, titania or a magnesia. Silica, which has a low thermal conductivity and can be easily controlled in the hydrogenation reaction, may be used as the support 5. It is noted that the shape of the support 5 illustrated in FIG. 1(a) is merely schematic, and the support 5 may be in the shape of, for example, a particle.

If the hydrogenation catalyst further comprises the support 5, the amount of the active metal element supported in the hydrogenation catalyst may be 5 to 60% by mass, or 5.57 to 9.61% by mass based on the total mass of the hydrogenation catalyst. If the hydrogenation catalyst further comprises the support 5, the amount of the additional element supported in the hydrogenation catalyst may be more than 0% by mass and 60% by mass or less, and may be 3.00 to 24.60% by mass based on the total mass of the hydrogenation catalyst. If the hydrogenation catalyst further comprises the support 5, the amount of the active component supported in the hydrogenation catalyst may be 5 to 70% by mass, or 9.08 to 34.21% by mass based on the total mass of the hydrogenation catalyst. Incidentally, the amount of the active metal element supported and the amount of the additional element supported can be measured by, for example, an ICP mass analysis method, an atomic absorption analysis method or the like.

<Method for producing cyclic saturated hydrocarbons>

A method for producing a cyclic saturated hydrocarbon of the present embodiment comprises a step of hydrogenating an aromatic hydrocarbon in the presence of the above-described hydrogenation catalyst and hydrogen.

The aromatic hydrocarbon to be hydrogenated by the hydrogenation catalyst of the present embodiment is not especially limited, and may be, for example, at least one selected from the group consisting of benzene, toluene, xylene, ethylbenzene, tetralin, naphthalene, methylnaphthalene and ethylnaphthalene. Since the hydrogenation catalyst of the present embodiment can inhibit a carbon-carbon bond cleavage reaction, which is a structure-sensitive reaction, the aromatic hydrocarbon to be hydrogenated may be at least one selected from the group consisting of toluene, xylene, ethylbenzene, tetralin, methylnaphthalene and ethylnaphthalene.

A reaction mode of the hydrogenation may be, for example, a fixed bed mode, a moving bed mode or a fluidized bed mode. A reaction temperature of the hydrogenation of the aromatic hydrocarbon (a temperature of the hydrogenation catalyst in the reaction) may be 150 to 350°C. If the hydrogenation of the aromatic hydrocarbon is a gas phase reaction performed under pressure of 0 to 10 MPa (gauge pressure), the amount of the aromatic hydrocarbon supplied to the hydrogenation catalyst per unit time may be 0.005 to 0.5 mL/min per 1 mL of the hydrogenation catalyst. A molar ratio of the hydrogen/aromatic hydrocarbon supplied to the hydrogenation catalyst may be 3 to 30.

<Method for producing hydrogenation catalyst for aromatic hydrocarbons>

If the hydrogenation catalyst of the present embodiment contains the active component and the support on which the active component is supported, the hydrogenation catalyst is produced by a coprecipitation method or a sequential impregnation method described below.

In employing the coprecipitation method, for example, an aqueous solution containing a salt of the active metal element (an active metal element solution) and an aqueous solution containing a salt of the additional element (an additional element solution) are first respectively prepared.

If the active metal element is nickel, the salt of the active metal element may be, for example, a water-soluble salt such as nickel sulfate or nickel nitrate. If the active metal element is palladium, the salt of the active metal element may be, for example, a water-soluble salt such as palladium nitrate or palladium chloride. If the active metal element is platinum, the salt of the active metal element may be, for example, a water-soluble salt such as chloroplatinic acid, tetraammine platinum hydroxide salt, tetraammine platinum nitrate, or bis(ethanolammonium)hexahydroxo platinate (IV). The active metal element solution can be prepared by, for example, adding the salt of the active metal element to water at a prescribed ratio, and mixing and stirring the resultant.

If the additional element is tin, the salt of the additional element may be, for example, a water-soluble salt such as tin sulfate or tin chloride. If the additional element is germanium, the salt of the additional element may be, for example, a water-soluble salt such as germanium chloride. If the additional element is gallium, the salt of the additional element may be, for example, a water-soluble salt such as gallium sulfate. If the additional element is copper, the salt of the additional element may be, for example, a water-soluble salt such as copper nitrate or copper sulfate. If the additional element is iron, the salt of the additional element may be, for example, a water-soluble salt such as iron nitrate or iron sulfate. The additional element solution can be prepared by, for example, adding the salt of the additional element to water at a prescribed ratio, and mixing and stirring the resultant.

A mixed salt solution is prepared by mixing the active metal element solution and the additional element solution so that a molar ratio between the active metal element and the additional element to be supported on the support can be a prescribed value. The support is added to the mixed salt solution. A neutralizer is added to the mixed salt solution containing the support for coprecipitating the active metal element and the additional element on the support, so as to obtain a coprecipitate containing the active metal element, the additional element and the support. The neutralizer may be, for example, sodium carbonate, sodium hydroxide, aqueous ammonia or urea. The pH of the mixed salt solution may be adjusted to 6 to 10 by adding the neutralizer.

The coprecipitate is dried and then heat treated to obtain a precursor of the active component. The coprecipitate may be washed with water before drying. A method for drying the coprecipitate may be, for example, vacuum drying or forced air drying. A heat treatment temperature for the coprecipitate may be, for example, 150 to 650°C. After forming the precursor by heat treating the coprecipitate in the air, the precursor is heated at 200 to 650°C under a hydrogen atmosphere. As a result, the precursor of the active component is reduced to generate the active component.

Through the coprecipitation method described above, the hydrogenation catalyst of the present embodiment can be obtained.

In employing the sequential impregnation method, for example, an aqueous solution containing a salt of the active metal element (an active metal element solution) is prepared so that the active metal element to be supported on the support can attain a prescribed mole number. The active metal element solution is added to the support for impregnating the active metal element into the support to obtain an active metal element impregnated product. The salt of the active metal element may be the same as that used in the coprecipitation method.

The active metal element impregnated product is dried and then heat treated to obtain an active metal element supported product. The active metal element impregnated product may be washed with water before drying. A method for drying the active metal element impregnated product may be, for example, the vacuum drying or the forced air drying. A heat treatment temperature for the active metal element impregnated product after the drying may be, for example, 150 to 650°C. The active metal element supported product may be obtained by heating the active metal element impregnated product at 200 to 650°C under a hydrogen atmosphere after performing the heat treatment in the air.

An aqueous solution containing a salt of the additional element (an additional element solution) is prepared. The additional element solution is added to the active metal element supported product so that a molar ratio between the active metal element and the additional element to be supported on the support can be a prescribed value, and thus, the additional element is impregnated into the active metal element supported product to obtain an additional element impregnated product. The salt of the additional element may be the same as that used in the coprecipitation method.

The additional element impregnated product is dried and then heat treated to obtain a precursor of the active component. The additional element impregnated product may be washed with water before drying. A method for drying the additional element impregnated product may be, for example, the vacuum drying or the forced air drying. A heat treatment temperature for the additional element impregnated product may be, for example, 150 to 650°C. After forming the precursor by heat treating the additional element impregnated product in the air, the precursor is heated at 200 to 650° under a hydrogen atmosphere. As a result, the precursor of the active component is reduced to generate the active component.

Through the sequential impregnation method described above, the hydrogenation catalyst of the present embodiment can be obtained.

EXAMPLES

The contents of the present invention will now be described in more details with reference to examples and comparative examples, and it is noted that the present invention is not limited to the examples described below.

(Example 1)

Silica was used as a support. A nickel sulfate aqueous solution and a tin sulfate aqueous solution were mixed to prepare a mixed salt solution. A molar ratio between nickel and tin in the mixed salt solution was adjusted to 3:1. The silica was added to the mixed salt solution. To the mixed salt solution containing the silica, a sodium carbonate aqueous solution was added as a neutralizer to adjust the pH of the mixed salt solution to 9, and thus, a coprecipitate containing nickel sulfate, tin sulfate and the support was obtained. After drying the coprecipitate, the resultant was calcined at 450°C under an air atmosphere. The thus obtained precursor of an active component was heat treated at 400°C under a hydrogen atmosphere to obtain a hydrogenation catalyst A-1. The amount of the nickel supported in the hydrogenation catalyst A-1 was 9.37% by mass based on the total mass of the hydrogenation catalyst A-1. The amount of the tin supported in the hydrogenation catalyst A-1 was 5.78% by mass based on the total mass of the hydrogenation catalyst A-1. Besides, a ratio of a mole number M2 of the tin to a total mole number (M1 + M2) of the active component of the hydrogenation catalyst A-1 (M2/(M1 + M2)) was 0.23.

(Example 2)

A hydrogenation catalyst A-2 was obtained in the same manner as in Example 1 except that a mixed salt solution was prepared so as to attain a molar ratio of 3:2 between the active metal element (nickel) and the additional element (tin) to be supported on silica. The amount of the nickel supported in the hydrogenation catalyst A-2 was 9.32% by mass based on the total mass of the hydrogenation catalyst A-2. The amount of the tin supported in the hydrogenation catalyst A-2 was 11.58% by mass based on the total mass of the hydrogenation catalyst A-2. Besides, M2/(M1 + M2) of the hydrogenation catalyst A-2 was 0.38.

(Example 3)

A hydrogenation catalyst A-3 was obtained in the same manner as in Example 1 except that a mixed salt solution was prepared so as to attain a molar ratio of 3:4 between nickel and tin to be supported on silica. The amount of the nickel supported in the hydrogenation catalyst A-3 was 9.61% by mass based on the total mass of the hydrogenation catalyst A-3. The amount of the tin supported in the hydrogenation catalyst A-3 was 24.60% by mass based on the total mass of the hydrogenation catalyst A-3. Besides, M2/(M1 + M2) of the hydrogenation catalyst A-3 was 0.56.

(Example 4)

A hydrogenation catalyst A-4 was obtained in the same manner as in Example 1 except that a mixed salt solution was prepared so as to attain a molar ratio of 6:1 between nickel and tin to be supported on silica. The amount of the nickel supported in the hydrogenation catalyst A-4 was 9.54% by mass based on the total mass of the hydrogenation catalyst A-4. The amount of the tin supported in the hydrogenation catalyst A-4 was 3.00% by mass based on the total mass of the hydrogenation catalyst A-4. Besides, M2/(M1 + M2) of the hydrogenation catalyst A-4 was 0.13.

(Example 5)

A hydrogenation catalyst A-5 was obtained in the same manner as in Example 1 except that the amounts of nickel and tin supported were adjusted by adjusting concentrations of a nickel salt and a tin salt in a mixed salt solution to be lower than in Example 1. The amount of the nickel supported in the hydrogenation catalyst A-5 was 5.57% by mass based on the total mass of the hydrogenation catalyst A-5. The amount of the tin supported in the hydrogenation catalyst A-5 was 3.51% by mass based on the total mass of the hydrogenation catalyst A-5. Besides, M2/(M1 + M2) of the hydrogenation catalyst A-5 was 0.24.

(Example 6)

Silica was used as a support. First, a nickel nitrate aqueous solution containing a prescribed amount of nickel based on the silica was prepared. The silica was impregnated with the nickel nitrate aqueous solution. The thus obtained nickel impregnated product was dried and then calcined at 450°C under an air atmosphere. The thus obtained calcined product was heat treated at 400°C under a hydrogen atmosphere to obtain a nickel supported product. Subsequently, the nickel supported product was impregnated with a tin chloride solution prepared to attain a molar ratio of 3:1 between nickel and tin to be supported on the silica. The thus obtained tin impregnated product was dried and then calcined at 450°C under an air atmosphere. The thus obtained precursor of an active component was heat treated at 400°C under a hydrogen atmosphere to obtain a hydrogenation catalyst A-6.

The amount of the nickel supported in the hydrogenation catalyst A-6 was 8.58% by mass based on the total mass of the hydrogenation catalyst A-6. The amount of the tin supported in the hydrogenation catalyst A-6 was 5.73% by mass based on the total mass of the hydrogenation catalyst A-6. Besides, M2/(M1 + M2) of the hydrogenation catalyst A-6 was 0.25.

(Example 7)

A hydrogenation catalyst A-7 was obtained in the same manner as in Example 6 except that amounts of a nickel nitrate aqueous solution and a tin chloride solution were adjusted so as to attain a molar ratio of 3:2 between nickel and tin to be supported on silica. The amount of the nickel supported in the hydrogenation catalyst A-7 was 8.11% by mass based on the total mass of the hydrogenation catalyst A-7. The amount of the tin supported in the hydrogenation catalyst A-7 was 11.09% by mass based on the total mass of the hydrogenation catalyst A-7. Besides, M2/(M1 + M2) of the hydrogenation catalyst A-7 was 0.40.

(Comparative Example 1)

As a hydrogenation catalyst B-1, a commercially available catalyst N-5256 manufactured by N. E. Chemcat Corporation was used. The hydrogenation catalyst B-1 was one prepared by the coprecipitation method. The amount of supported nickel in the hydrogenation catalyst B-1 was 57% by mass based on the total mass of the hydrogenation catalyst B-1. The amount of tin supported in the hydrogenation catalyst B-1 was 0% by mass based on the total mass of the hydrogenation catalyst B-1.

(Comparative Example 2)

Silica was used as a support. First, a nickel nitrate aqueous solution containing a prescribed amount of nickel based on the silica was prepared. The silica was impregnated with the nickel nitrate aqueous solution. The thus obtained nickel impregnated product was dried and then calcined at 450°C under an air atmosphere. The thus obtained precursor of an active component was heat treated at 400°C under a hydrogen atmosphere to obtain a hydrogenation catalyst B-2. The amount of the nickel supported in the hydrogenation catalyst B-2 was 9.74% by mass based on the total mass of the hydrogenation catalyst B-2. The amount of tin supported in the hydrogenation catalyst B-2 was 0% by mass based on the total mass of the hydrogenation catalyst B-2.

(Comparative Example 3)

Silica was used as a support. A nickel sulfate aqueous solution containing a prescribed amount of nickel based on the silica was prepared, and the silica was added to the nickel sulfate aqueous solution. To the nickel sulfate aqueous solution containing the silica, a sodium carbonate aqueous solution was added as a neutralizer to adjust the pH of a mixed salt solution to 9, and thus, a precipitate containing nickel sulfate and the support was obtained. The precipitate was dried and then calcined at 450°C under an air atmosphere. The thus obtained precursor of an active component was heat treated at 400° under a hydrogen atmosphere to obtain a hydrogenation catalyst B-3. The amount of the nickel supported in the hydrogenation catalyst B-3 was 10% by mass based on the total mass of the hydrogenation catalyst B-3. The amount of tin supported in the hydrogenation catalyst B-3 was 0% by mass based on the total mass of the hydrogenation catalyst B-3.

[Measurement of X-ray diffraction]

XRD spectra of the hydrogenation catalysts A-1 to A-7 and the hydrogenation catalyst B-3 were respectively measured under the following conditions:

Diffractometer: RINT 2500 (manufactured by Rigaku Corporation)
X-ray source: CuK$\alpha$ (with a monochromator used)
Tube voltage: 50 kV
Tube current: 200 mA
Divergence slit: 1/2°
Scattering slit: 1/2°
Light receiving slit: 0.15 mm
Diffraction angle 2$\theta$: 5 to 90°

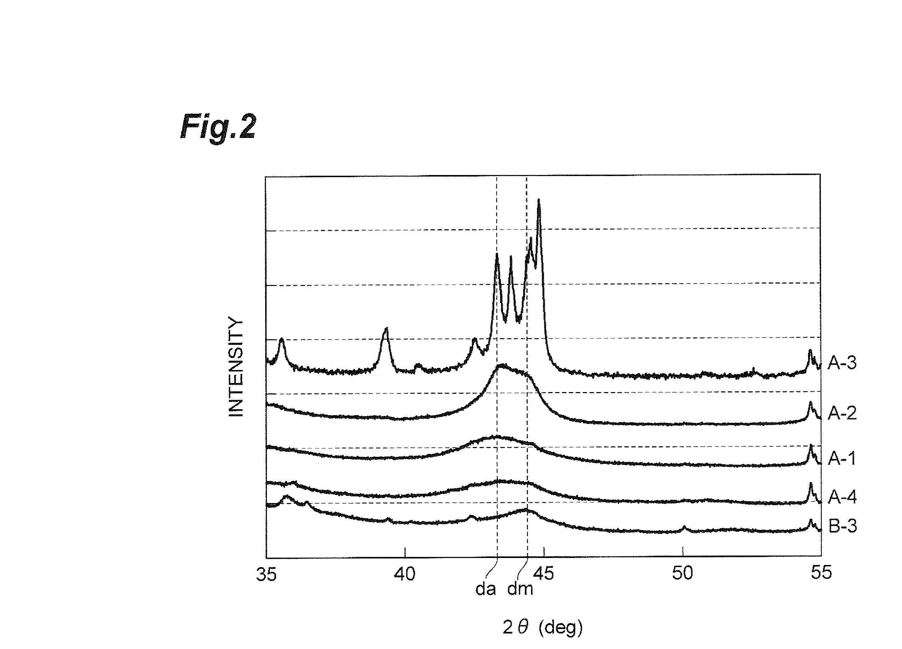

The XRD spectra of the hydrogenation catalysts A-1 to A-4 and the hydrogenation catalyst B-3 obtained in a range of the diffraction angle of 35 to 55° are illustrated in FIG. 2. It was confirmed that the XRD spectrum of the hydrogenation catalyst B-3 has a local maximum value at a diffraction angle dm (of approximately 44.5°). An X-ray diffraction intensity Im of the hydrogenation catalyst B-3 at the diffraction angle dm (of approximately 44.5°) is derived from the crystal structure of a simple substance m (elemental nickel). On the other hand, it was confirmed that the XRD spectrum of each of the hydrogenation catalysts A-1 to A-4 has a local maximum value at a diffraction angle da (of approximately 43.5°) different from the diffraction angle dm (of approximately 44.5°). It was also confirmed that an X-ray diffraction intensity Ia at the diffraction angle da (of approximately 43.5°) in the XRD spectrum of each of the hydrogenation catalysts A-1, A-2 and A-4 was larger than an X-ray diffraction intensity Im at the diffraction angle dm (of approximately 44.5°). It was confirmed that the XRD spectrum of the hydrogenation catalyst A-3 has a plurality of local maximum values not only at approximately 43.5° but also at a plurality of diffraction angles da. It was confirmed that the XRD spectrum of the hydrogenation catalyst A-5 also has a local maximum value at a diffraction angle da (of approximately 43.5°) different from the diffraction angle dm (of approximately 44.5°). It was confirmed that the XRD spectrum of each of the hydrogenation catalysts A-6 and A-7 has a local maximum value at a diffraction angle da (of approximately 30.4°) different from the diffraction angle dm (of approximately 44.5°).

[Analysis with scanning transmission electron microscope]

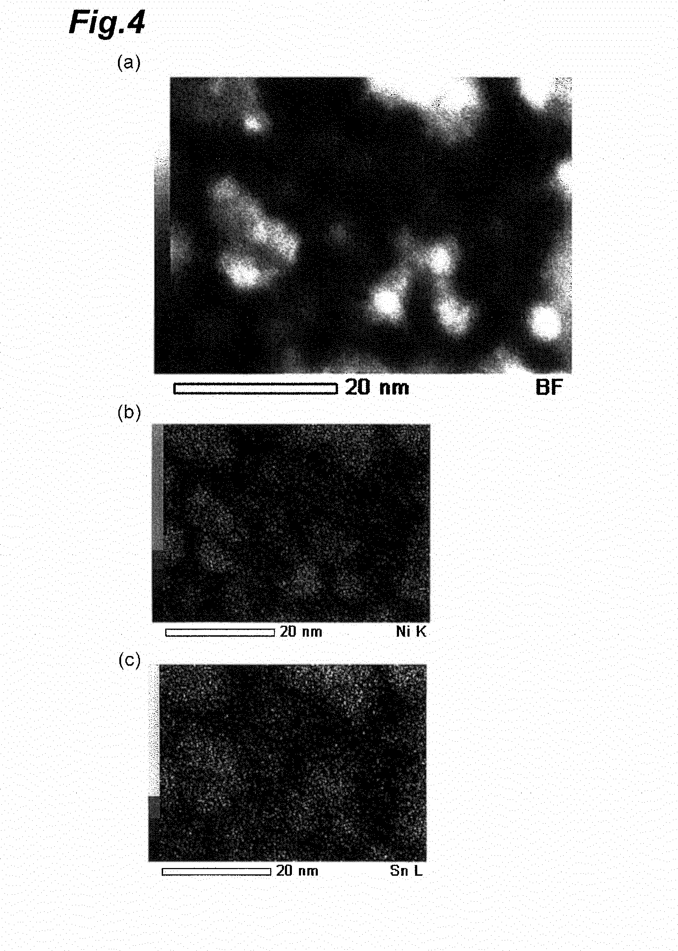

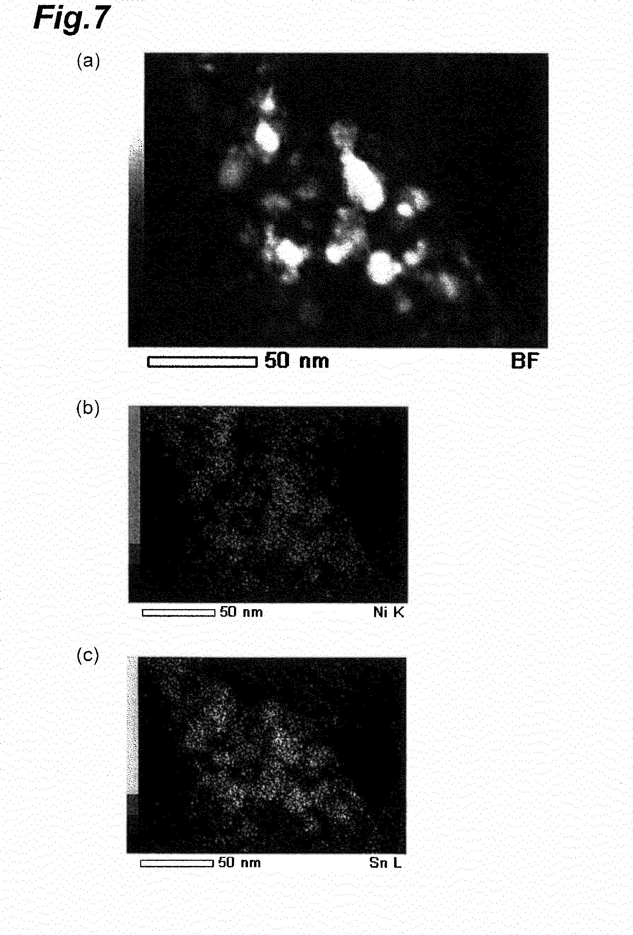

The hydrogenation catalysts A-1 and A-2 were analyzed with a scanning transmission electron microscope as described below. Three portions on the surface of each hydrogenation catalyst were analyzed. The analysis results of the hydrogenation catalyst A-1 are illustrated in FIG. 4 to 6. The analysis results of the hydrogenation catalyst A-2 are illustrated in FIG. 7 to 9.

An image of one given portion of the hydrogenation catalyst A-1 taken by high-angle annular dark-field scanning transmission electron microscopy (HAADF-STEM) is illustrated in FIG. 4(a). Results of area analysis (elemental mapping) of the portion illustrated in FIG. 4(a) are illustrated in FIG. 4(b) and 4(c). Specifically, FIG. 4(a), 4(b) and 4(c) illustrate the same portion of the hydrogenation catalyst A-1. The area analysis was performed by using an energy dispersive X-ray spectrometer (STEM-EDS) attached to the STEM. A pale (white) spot in FIG. 4(a) corresponds to a place where there is an active site containing the active component (nickel and tin). As illustrated in FIG. 4(a), it was confirmed that a plurality of particulate active sites having a longest diameter smaller than 20 nm are dispersed on the surface of the hydrogenation catalyst A-1. A pale (white) spot in FIG. 4(b) corresponds to a place where there is nickel. A pale (white) spot in FIG. 4(c) corresponds to a place where there is tin. As illustrated in FIG. 4(a), 4(b) and 4(c), it was confirmed that every minute active site which the hydrogenation catalyst A-1 has is an alloy containing nickel and tin, and that atoms or molecules of the nickel and the tin are highly dispersed on the surface of the active site.

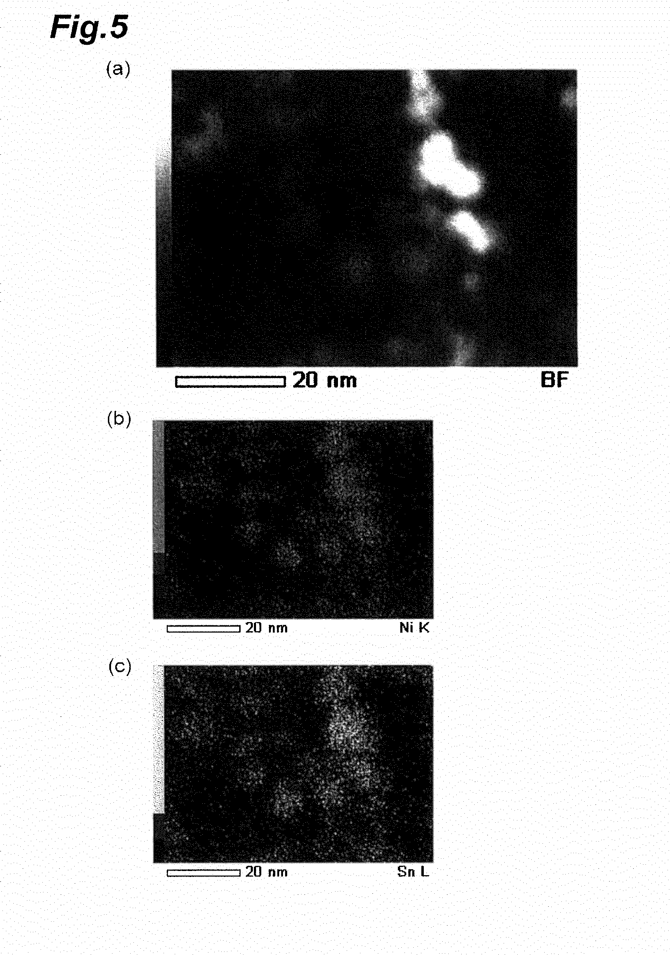

An image of one portion of the hydrogenation catalyst A-1 taken by the HAADF-STEM is illustrated in FIG. 5(a). The portion illustrated in FIG. 5(a) is different from the portion illustrated in FIG. 4(a). Results of the area analysis (elemental mapping) of the portion illustrated in FIG. 5(a) are illustrated in FIG. 5(b) and 5(c). Specifically, FIG. 5(a), 5(b) and 5(c) illustrate the same portion of the hydrogenation catalyst A-1. A pale (white) spot in FIG. 5(a) corresponds to a place where there is an active site containing the active component (nickel and tin). As illustrated in FIG. 5(a), it was confirmed that a plurality of particulate active sites having a longest diameter smaller than 20 nm are dispersed on the surface of the hydrogenation catalyst A-1. A pale (white) spot in FIG. 5(b) corresponds to a place where there is nickel. A pale (white) spot in FIG. 5(c) corresponds to a place where there is tin. As illustrated in FIG. 5(a), 5(b) and 5(c), it was confirmed that every minute active site which the hydrogenation catalyst A-1 has is an alloy containing nickel and tin, and that atoms or molecules of the nickel and the tin are highly dispersed on the surface of the active site.

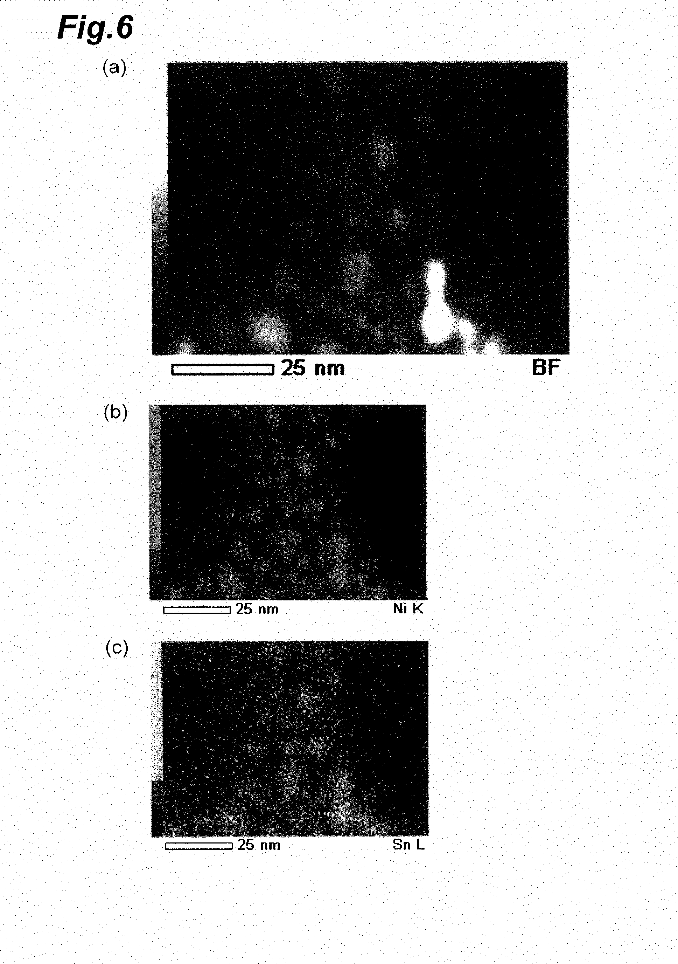

An image of one portion of the hydrogenation catalyst A-1 taken by the HAADF-STEM is illustrated in FIG. 6(a).

The portion illustrated in FIG. 6(a) is different from the portions illustrated in FIG. 4(a) and 5(a). Results of the area analysis (elemental mapping) of the portion illustrated in FIG. 6(a) are illustrated in FIG. 6(b) and 6(c). Specifically, FIG. 6(a), 6(b) and 6(c) illustrate the same portion of the hydrogenation catalyst A-1. A pale (white) spot in FIG. 6(a) corresponds to a place where there is an active site containing the active component (nickel and tin). As illustrated in FIG. 6(a), it was confirmed that a plurality of particulate active sites having a longest diameter smaller than 25 nm are dispersed on the surface of the hydrogenation catalyst A-1. A pale (white) spot in the FIG. 6(b) corresponds to a place where there is nickel. A pale (white) spot in FIG. 6(c) corresponds to a place where there is tin. As illustrated in FIG. 6(a), 6(b) and 6(c), it was confirmed that every minute active site which the hydrogenation catalyst A-1 has is an alloy containing nickel and tin, and that atoms or molecules of the nickel and the tin are highly dispersed on the surface of the active site.

An image of one given portion of the hydrogenation catalyst A-2 taken by the HAADF-STEM is illustrated in FIG. 7(a). Results of the area analysis (elemental mapping) of the portion illustrated in FIG. 7(a) are illustrated in FIG. 7(b) and 7(c). Specifically, FIG. 7(a), 7(b) and 7(c) illustrate the same portion of the hydrogenation catalyst A-2. A pale (white) spot in FIG. 7(a) corresponds to a place where there is an active site containing the active component (nickel and tin). As illustrated in FIG. 7(a), it was confirmed that a plurality of particulate active sites having a longest diameter smaller than 50 nm are dispersed on the surface of the hydrogenation catalyst A-2. A pale (white) spot in FIG. 7(b) corresponds to a place where there is nickel. A pale (white) spot in FIG. 7(c) corresponds to a place where there is tin. As illustrated in FIG. 7(a), 7(b) and 7(c), it was confirmed that every minute active site which the hydrogenation catalyst A-2 has is an alloy containing nickel and tin, and that atoms or molecules of the nickel and the tin are highly dispersed on the surface of the active site.

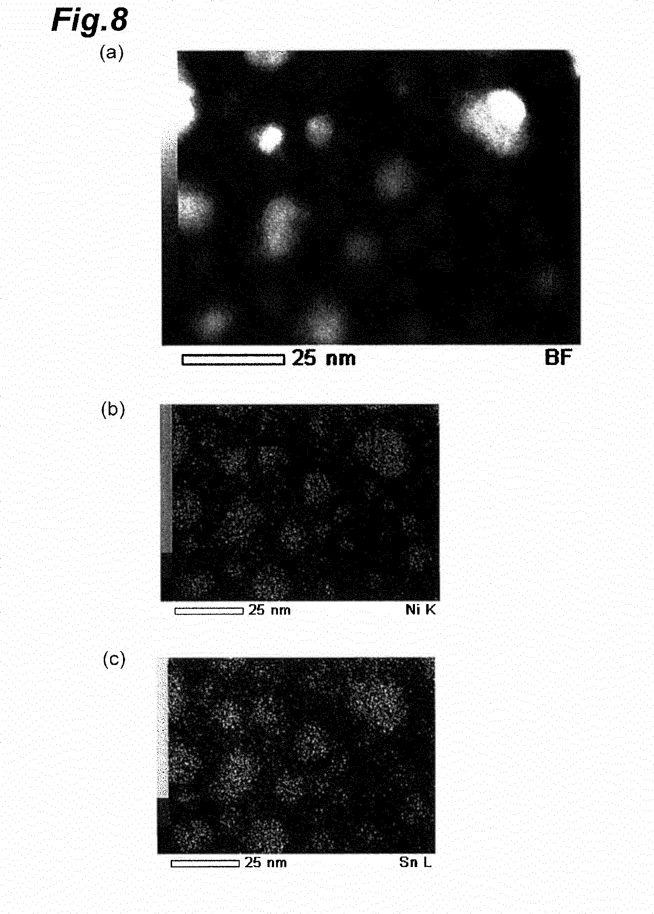

An image of one portion of the hydrogenation catalyst A-2 taken by the HAADF-STEM is illustrated in FIG. 8(a). The portion illustrated in FIG. 8(a) is different from the portion illustrated in FIG. 7(a). Results of the area analysis (elemental mapping) of the portion illustrated in FIG. 8(a) are illustrated in FIG. 8(b) and 8(c). Specifically, FIG. 8(a), 8(b) and 8(c) illustrate the same portion of the hydrogenation catalyst A-2. A pale (white) spot in FIG. 8(a) corresponds to a place where there is an active site containing the active component (nickel and tin). As illustrated in FIG. 8(a), it was confirmed that a plurality of particulate active sites having a longest diameter smaller than 25 nm are dispersed on the surface of the hydrogenation catalyst A-2. A pale (white) spot in FIG. 8(b) corresponds to a place where there is nickel. A pale (white) spot in FIG. 8(c) corresponds to a place where there is tin. As illustrated in FIG. 8(a), 8(b) and 8(c), it was confirmed that every minute active site which the hydrogenation catalyst A-2 has is an alloy containing nickel and tin, and that atoms or molecules of the nickel and the tin are highly dispersed on the surface of the active site.

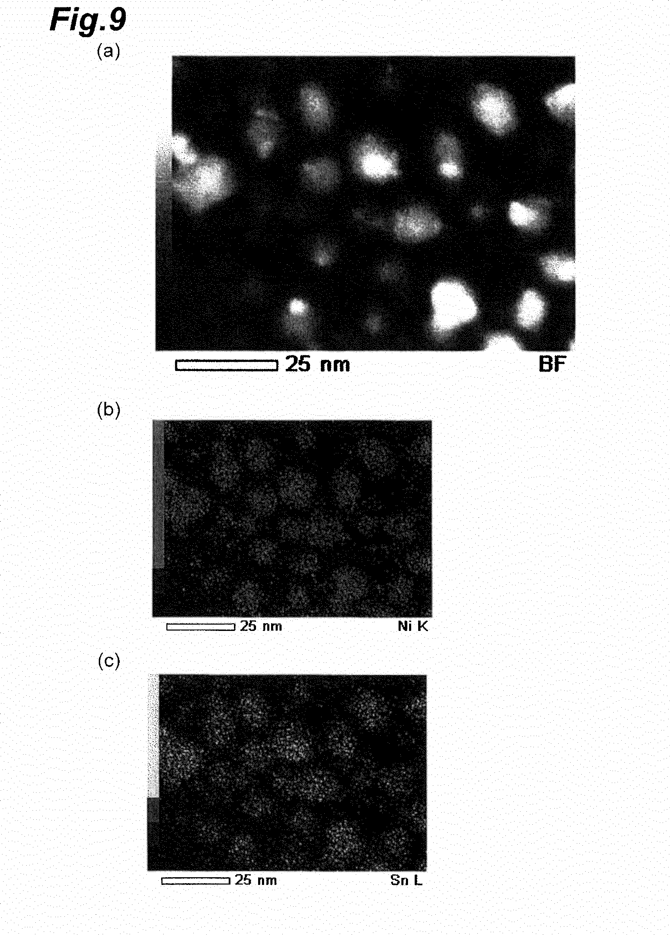

An image of one portion of the hydrogenation catalyst A-2 taken by the HAADF-STEM is illustrated in FIG. 9(a). The portion illustrated in FIG. 9(a) is different from the portions illustrated in FIG. 7(a) and 8(a). Results of the area analysis (elemental mapping) of the portion illustrated in FIG. 9(a) are illustrated in FIG. 9(b) and 9(c). Specifically, FIG. 9(a), 9(b) and 9(c) illustrate the same portion of the hydrogenation catalyst A-2. A pale (white) spot in FIG. 9(a) corresponds to a place where there is an active site containing the active component (nickel and tin). As illustrated in FIG. 9(a), it was confirmed that a plurality of particulate active sites having a longest diameter smaller than 25 nm are dispersed on the surface of the hydrogenation catalyst A-2. A pale (white) spot in FIG. 9(b) corresponds to a place where there is nickel. A pale (white) spot in FIG. 9(c) corresponds to a place where there is tin. As illustrated in FIG. 9(a), 9(b) and 9(c), it was confirmed that every minute active site which the hydrogenation catalyst A-2 has is an alloy containing nickel and tin, and that atoms or molecules of the nickel and the tin are highly dispersed on the surface of the active site.

[Evaluation of hydrogenation activity]

Hydrogenation of toluene was performed as follows:

Alumina, 3 mL of the hydrogenation catalyst A-1 and alumina were stacked in this order in a fixed-bed flow reactor, so as to fill the reactor with the alumina and the hydrogenation catalyst A-1. As a pretreatment, a reduction treatment of the hydrogenation catalyst with hydrogen was performed at 250°C for 1 hour. After the pretreatment, vaporized toluene and a hydrogen gas were supplied into the reactor while retaining the temperature of the hydrogenation catalyst A-1 (the temperature at the center of the catalyst layer) at 250°C. Incidentally, pressure within the reactor was adjusted to 0.18 MPa (gauge pressure). The toluene was vaporized by heating the toluene prior to the supply to the reactor at 150°C in a vaporizer. The supply rate of the toluene was adjusted to 0.1 mL/min, and the space velocity (SV) of the toluene was adjusted to 2 h$^{-1}$. The mole number of the hydrogen supplied to the reactor was adjusted to six times as large as that of the toluene supplied to the reactor.

When five hours elapsed from the start of the reaction, a gas discharged from the reactor was collected and cooled to obtain a product oil. The product oil was analyzed by using a gas chromatography-flame ionization detector (GC-FID), so as to obtain a GC area (peak area) of toluene, a GC area of methylcyclohexane and a GC area of cyclohexane contained in the product oil. On the basis of these analysis results, a toluene conversion rate (unit: %)

defined by the following expression was calculated. The methylcyclohexane corresponds to a target product (an organic hydride), and the cyclohexane corresponds to a byproduct.

$$\text{Toluene conversion rate (\%)} = \{1 - (m1/m0)\} \times 100$$

wherein m1 represents a mole number of the toluene in the product oil, which has a value on the basis of the analysis performed by using the GC-FID; and m0 represents a mole number of the toluene supplied to the reactor.

On the basis of the GC area of the cyclohexane contained in the product oil, a cyclohexane concentration in the product oil was calculated. The result is shown in Table 1.

In the same manner as in the case where the hydrogenation catalyst A-1 was used, the hydrogenation of toluene was performed by using each of the hydrogenation catalysts A-2 to A-7 and the hydrogenation catalysts B-1 to B-3. No matter which of the hydrogenation catalysts of these examples and comparative examples was used, it was confirmed that methylcyclohexane was generated through the hydrogenation of toluene. The toluene conversion rate and the cyclohexane concentration in a product oil attained by using each of the hydrogenation catalysts were calculated. The results are shown in Tables 1 and 2. Incidentally, in the hydrogenation of toluene performed by using the hydrogenation catalyst B-1, the temperature of the hydrogenation catalyst B-1 was retained at 271°C, and the mole number of the hydrogen supplied to the reactor was adjusted to 4.5 times as large as the mole number of the toluene supplied to the reactor.

[Table 1]

| | Comparative example 1 | Comparative example 2 | Comparative example 3 | Example 1 | Example 2 | Example 3 |
|---|---|---|---|---|---|---|
| Hydrogenation catalyst | B-1 | B-2 | B-3 | A-1 | A-2 | A-3 |
| Active metal element | Ni | Ni | Ni | Ni | Ni | Ni |
| Additional element | - | - | - | Sn | Sn | Sn |
| Active metal element/additional element (Charged molar ratio) | - | - | - | 3/1 | 3/2 | 3/4 |
| Amount of active metal element supported (mass%) | 57 | 9.74 | 10 | 9.37 | 9.32 | 9.61 |
| Amount of additional element supported (mass%) | 0 | 0 | 0 | 5.78 | 11.58 | 24.60 |
| M2/M1 | - | - | - | 0.30 | 0.61 | 1.27 |
| M2/(M1+M2) | - | - | - | 0.23 | 0.38 | 0.56 |
| M1/M2 | - | - | - | 3.28 | 1.63 | 0.79 |
| Preparation method | Coprecipitation | Impregnation | Coprecipitation | Coprecipitation | Coprecipitation | Coprecipitation |
| Toluene conversion rate (250°C) (%) | 92.4 | 27.7 | 64.6 | 29.3 | 16.9 | 18.5 |
| Cyclohexane concentration (mol ppm) | 37900 | 139 | 385 | 11 | 0 | 0 |

[Table 2]

| | Example 4 | Example 5 | Example 6 | Example 7 |
|---|---|---|---|---|
| Hydrogenation catalyst | A-4 | A-5 | A-6 | A-7 |
| Active metal element | Ni | Ni | Ni | Ni |
| Additional element | Sn | Sn | Sn | Sn |
| Active metal element/additional element (Charged molar ratio) | 6/1 | 3/1 | 3/1 | 3/2 |
| Amount of active metal element supported (mass%) | 9.54 | 5.57 | 8.58 | 8.11 |
| Amount of additional element supported (mass%) | 3.00 | 3.51 | 5.73 | 11.09 |
| M2/M1 | 0.16 | 0.31 | 0.33 | 0.68 |
| M2/(M1+M2) | 0.13 | 0.24 | 0.25 | 0.40 |
| M1/M2 | 6.43 | 3.21 | 3.03 | 1.48 |
| Preparation method | Coprecipitation | Coprecipitation | Sequential impregnation | Sequential impregnation |
| Toluene conversion rate (250°C) (%) | 39.8 | 8.4 | 37.3 | 3.8 |
| Cyclohexane concentration (mol ppm) | 11 | 0 | 76 | 0 |

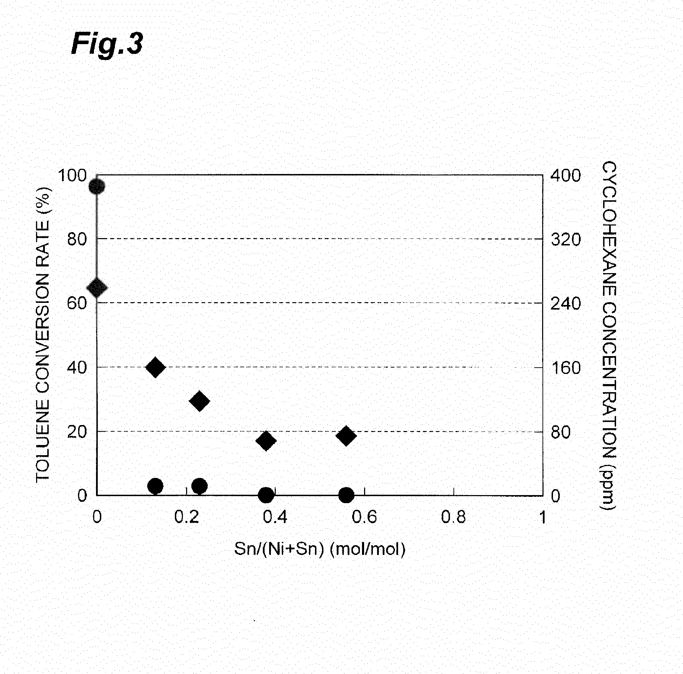

A relationship among a molar content of the tin in the active component of each of the hydrogenation catalysts A-1 to A-4 and the hydrogenation catalyst B-3, a toluene conversion rate in the hydrogenation reaction using each of these catalysts, and a cyclohexane concentration (unit: mol ppm) in the product oil is illustrated in FIG. 3. In FIG. 3, each black square indicates the toluene conversion rate, and each black circle indicates the cyclohexane concentration in the product oil. It is revealed that the cyclohexane concentration is extremely low and a carbon-carbon bond cleavage reaction is inhibited when the hydrogenation catalysts A-1 to A-4 are used. As shown in Table 2, also when the hydrogenation catalysts A-5 to A-7 were used, it was confirmed that the cyclohexane concentration in the product oil was low.

On the other hand, when the hydrogenation catalyst B-3 was used, although the toluene conversion rate was high, the cyclohexane concentration was also high. As shown in Table 1, the cyclohexane concentrations in the product oils obtained by the hydrogenation reaction using the hydrogenation catalysts B-1 and B-2 were also high.

REFERENCE SIGNS LIST

hydrogenation catalyst, 2, 8 ... active metal element, 4 ... additional element, 3, 5 ... support, 6, 7 ... active site, 10 ... conventional hydrogenation catalyst

**Claims**

1. A hydrogenation catalyst for an aromatic hydrocarbon, comprising an active component containing an active metal element and an additional element,
   wherein the active metal element is one selected from the group consisting of nickel, palladium and platinum,
   the additional element is one selected from the group consisting of tin, germanium, gallium, copper and iron, and
   an X-ray diffraction spectrum of the active component has a local maximum value at a diffraction angle da different from the diffraction angle dm, where a diffraction angle of diffracted X-ray derived from a crystal structure of a simple substance m of the active metal element is dm.

2. The hydrogenation catalyst according to claim 1,
   wherein M1 and M2 satisfy the following expression (1):

$$0.01 \leq M2/(M1 + M2) \leq 0.6 \quad (1)$$

where a mole number of the active metal element contained in the active component is M1 and a mole number of the additional element contained in the active component is M2.

3. The hydrogenation catalyst according to claim 1 or 2, wherein the active metal element is nickel, and the additional element is tin.

4. The hydrogenation catalyst according to any one of claims 1 to 3, further comprising silica on which the active component is supported.

5. A method for producing a cyclic saturated hydrocarbon, comprising a step of hydrogenating an aromatic hydrocarbon in the presence of the hydrogenation catalyst according to any one of claims 1 to 4 and hydrogen.

# Fig.1

(a)

(b)

EP 2 929 937 A1

## Fig.2

**Fig.3**

# Fig.4

(a)

20 nm      BF

(b)

20 nm      Ni K

(c)

20 nm      Sn L

# Fig.5

(a)

20 nm · · · · · BF

(b)

20 nm · · · · · Ni K

(c)

20 nm · · · · · Sn L

# Fig.6

(a)

25 nm                    BF

(b)

25 nm          Ni K

(c)

25 nm          Sn L

*Fig.7*

(a)

50 nm                    BF

(b)

50 nm                    Ni K

(c)

50 nm                    Sn L

## Fig.8

(a)

25 nm      BF

(b)

25 nm      Ni K

(c)

25 nm      Sn L

**Fig.9**

(a)

25 nm    BF

(b)

25 nm    Ni K

(c)

25 nm    Sn L

Europäisches
Patentamt

European
Patent Office

Office européen
des brevets

# EUROPEAN SEARCH REPORT

Application Number

EP 15 16 1805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2011/166398 A1 (FISCHER LARS [FR] ET AL) 7 July 2011 (2011-07-07) * paragraphs [0002] - [0003], [0008], [0010] - [0011], [0019], [0027] - [0028], [0033], [0035] * * examples 3, 6 * * paragraph [0065]; tables 1-2 * | 1-5 | INV. B01J23/835 B01J23/825 B01J23/755 B01J23/89 B01J35/00 B01J37/03 B01J37/02 B01J37/08 B01J37/18 |
| X | US 3 480 531 A (MULASKEY BERNARD F) 25 November 1969 (1969-11-25) * column 1, lines 15-20, 59-60 * * column 2, lines 9-15,29-34,39-49, 69-70 * * column 3, lines 3-10, 32-40 * * column 4, lines 58-59 * * column 5, lines 11-17, 22-23, 35-48, 61-66, 73-75 * * column 6, lines 1-15, 45-49, 52-55 * | 1-5 | ADD. C10G45/52 C10G45/48 C07C5/10 |
| X | US 4 251 394 A (CARTER JAMES L ET AL) 17 February 1981 (1981-02-17) * column 4, lines 31-35, 45-48 * * examples 1, 4 * | 1-5 | TECHNICAL FIELDS SEARCHED (IPC) B01J C07C C10G |
| X | US 2013/184507 A1 (GIEDIGKEIT RAINER [DE] ET AL) 18 July 2013 (2013-07-18) * paragraphs [0026], [0055], [0068]; figure 2 * | 1-5 | |

The present search report has been drawn up for all claims

| Place of search | Date of completion of the search | Examiner |
|---|---|---|
| Munich | 25 August 2015 | Marchand, Karin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 15 16 1805

This annex lists the patent family members relating to the patent documents cited in the above-mentioned European search report.
The members are as contained in the European Patent Office EDP file on
The European Patent Office is in no way liable for these particulars which are merely given for the purpose of information.

25-08-2015

| Patent document cited in search report | | Publication date | Patent family member(s) | | Publication date |
|---|---|---|---|---|---|
| US 2011166398 | A1 | 07-07-2011 | CN | 101992090 A | 30-03-2011 |
| | | | DK | 2287274 T3 | 02-04-2013 |
| | | | EP | 2287274 A1 | 23-02-2011 |
| | | | FR | 2949078 A1 | 18-02-2011 |
| | | | JP | 5514668 B2 | 04-06-2014 |
| | | | JP | 2011036858 A | 24-02-2011 |
| | | | US | 2011166398 A1 | 07-07-2011 |
| US 3480531 | A | 25-11-1969 | NONE | | |
| US 4251394 | A | 17-02-1981 | NONE | | |
| US 2013184507 | A1 | 18-07-2013 | CA | 2646099 A1 | 20-09-2007 |
| | | | CN | 101400631 A | 01-04-2009 |
| | | | EP | 1834939 A1 | 19-09-2007 |
| | | | EP | 2004577 A1 | 24-12-2008 |
| | | | ES | 2398250 T3 | 14-03-2013 |
| | | | JP | 5302693 B2 | 02-10-2013 |
| | | | JP | 2009529560 A | 20-08-2009 |
| | | | MY | 146921 A | 15-10-2012 |
| | | | SG | 172714 A1 | 28-07-2011 |
| | | | US | 2009221861 A1 | 03-09-2009 |
| | | | US | 2013184507 A1 | 18-07-2013 |
| | | | WO | 2007104569 A1 | 20-09-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002542928 A